# EUROPEAN PATENT SPECIFICATION

(11) **EP 0 666 847 B1**
(45) Date of publication and mention of the grant of the patent: **17.06.1998**
(21) Application number: 94925317.3
(22) Date of filing: 31.08.1994
(51) Int. Cl.: C07D 209/16, A61K 31/40

(54) **TRYPTAMINE ANALOGS WITH 5-HT1D SELECTIVITY**
TRYPTAMIN ANALOGE MIT 5-HT1D SELEKTIVITÄT
ANALOGUES DE LA TRYPTAMINE AYANT UNE SELECTIVITE AU 5-HT1D

(30) Priority: 01.09.1993 US 115003
(43) Date of publication of application: 16.08.1995
(73) Proprietor: Allelix Biopharmaceuticals Inc., Mississauga, Ontario, L4V 1V7 (CA); VIRGINIA COMMONWEALTH UNIVERSITY, Richmond, VA 23298-0568 (US)
(72) Inventor: GLENNON, Richard, A., Richmond, VA 23235 (US)
(74) Representative: Eyles, Christopher Thomas
(86) International application number: CA9400476
(87) International publication number: WO9506638

(56) References cited:
- WO-A-92/14708
- GB-A- 2 186 874
- US-A- 3 317 560
- CHEMICAL ABSTRACTS, vol. 80, no. 13, 1 April 1974, Columbus, Ohio, US; abstract no. 70637p, SUVOROV N.N. ET AL. 'Indole derivatives.LXXXVIII. Synthesis ...' & KHIM. GETEROTSIKL. SOEDIN. 1973,(11),1515-18
- JOURNAL OF MEDICINAL CHEMISTRY, vol.36, no.11, 28 May 1993, WASHINGTON US pages 1529 - 1538 L.J. STREET ET AL. 'Synthesis and serotonergic activity of 5-(oxadiazolyl)tryptamines: potent agonists for 5-HT1D receptors'

## Description

This invention relates to tryptamine analogs having 5-HT receptor binding activity, and to their production and use.

### Background to the Invention

Through its interaction with receptors borne on neuronal and other cells, 5-hydroxytryptamine (5-HT; serotonin), exerts various physiological effects. Imbalances in this interaction are believed responsible for such conditions as anxiety, depression, hallucination, migraine, chemotherapy-induced nausea, and for disorders in sexual activity, cardiovascular activity and thermoregulation, among others. From an improved understanding of the 5-HT receptor population, it is apparent that these effects are mediated selectively through individual types and sub-types of 5-HT receptors.

Migraine, for example, has been treated with ergotamine, dihydroergotamine and methysergide, all of which act at 5-HT1 type receptors. Their use is associated with undesirable side effects, however, likely because they interact also with various other 5-HT receptor types. An improved side effect profile is seen with the more recently marketed tryptamine analog known as sumatriptan, which binds somewhat selectively at the 5-HT1 receptor sub-type known as 5-HT1D (see Glennon and Westkaemper, DN&P, 1993, 6(6):390).

Tryptamines having 5-HT receptor binding activity have previously been described. For example, WO-A-92/14708 reports typtamines with a sulfonamido substituent at the 5-position of the indole ring. Tryptamines with a heterocyclic group at the 5-position of the indole ring are disclosed in J. Med. Chem. 1993, 36:1529-1538. The radioprotective properties of optionally substituted 5-alkoxytryptamines having chain lengths of 5 carbon atoms or less are described in Khim. Geterotsikl. Soedin. 1973, (11); 1515-1518 (see Chemical Abstracts, Vol. 80, No. 13, 1 April 1974, Abstract No. 70637P).

Example 7(b) of US-A-3317560 discloses the compound 5-octyloxy-3-(2-ethylaminoethyl) indole as an intermediate for the preparation of 1-ethyl-1-[2-(5-octyloxyindole-3-yl)-ethyl] guanidine hydrochloride.

Given the physiological and clinical significance of the 5-HT1D receptor, it would be desirable to provide compounds capable of binding tightly and selectively to this receptor, for medical use for example to treat indications such as migraine and others for which administration of a 5-HT1D ligand is indicated, and also for research and diagnostic use for example to identify these receptors and to screen for drug candidates.

### Summary of the Invention

The present invention provides the use for the preparation of a pharmaceutical composition of a tryptamine analog of formula (I) or a salt, solvate or hydrate thereof: 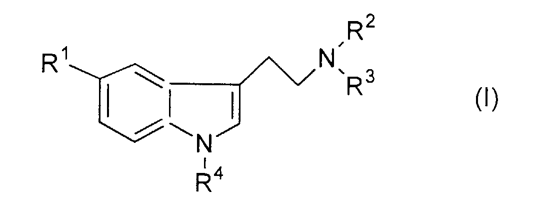 wherein
R¹ represents a chain selected from C₈₋₁₁alkyl, C₇₋₁₀alkoxy, C₈₋₁₁alkanoyl and C₇₋₁₀alkanoyloxy wherein said chain is optionally substituted by hydroxyl, C₁₋₄alkyl or C₁₋₄alkoxy and wherein one of the intervening carbons of said chain is optionally replaced with a heteroatom selected from oxygen, nitrogen and sulfur;
R² and R³ each independently represent H or C₁₋₃alkyl; and
R⁴ represents H or C₁₋₄alkyl, aryl or aryl C₁₋₄ alkyl.

In another of its aspects, the present invention provides processes for preparing compounds of formula (I) and intermediates useful in such processes.

In a further aspect, the present invention provides compositions containing compounds of formula (I) either for use as reagents for example in the identification of 5-HT1D receptors or in screening for ligands of such receptors, or for use pharmaceutically to treat conditions where a 5-HT1D ligand is indicated.

Moreover the invention also provides compounds of formula (I) with the proviso that, when R⁴ is hydrogen and one of R² and R³ is hydrogen and the other of R² and R³ is ethyl, then R¹ is not n-octyloxy.

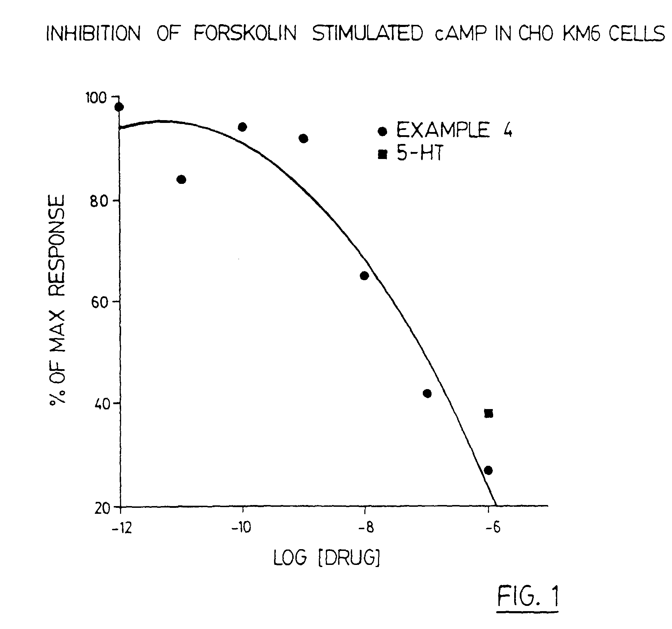

The invention is now described in greater detail with reference to the accompanying Figure 1, which illustrates the stimulatory effect of a compound of the invention on cells presenting the 5-HT1D receptor.

### Detailed Description of the Invention and Preferred Embodiments

The present invention provides tryptamine analogs that bind with relative high affinity and selectivity to the 5-HT1D receptor. Compounds having this desirable combination of properties are tryptamine analogs in which the 5-position is substituted by a group, designated R¹, having a length in the range from 8 to 11 atoms.

In particular and with reference to Formula I, the group R¹ can be selected from linear C₈₋₁₁alkyl, C₈₋₁₁alkanoyl, C₇₋₁₀alkoxy in which the ether oxygen is attached at the 5-position, and C₇₋₁₀alkanoyloxy in which the ester oxygen is attached at the 5-position. These groups suitably have a chain length of 8-11, and most suitably of 8, 9 or 10 atoms.

In specific embodiments of the invention, R¹ is C₇₋₁₀alkoxy, preferably C₇₋₉alkoxy and most preferably C₉alkoxy (nonyloxy).

The alkyl, alkoxy, alkanoyl or alkanoyloxy chain constituting R¹ can be substituted by one or more e.g. up to 4 substituents, suitably 1 or 2 substituents, selected from hydroxyl; linear or branched chain C₁₋₄alkyl such as methyl, ethyl, propyl (n- and i-), butyl (n-, i- and t-); and linear or branched chain C₁₋₄alkoxy such as methoxy, ethoxy, propoxy (n- and i-) and butoxy (n-, i- and t-). In specific embodiments, R¹ is the group 8-hydroxyoctyloxy or the group 7,7-dimethyloctyloxy.

A substituent may be conjugated to any one of the carbons within the carbon chain of the group constituting R¹. In selecting R¹ substituents, it is desirable to maintain the total chain length of R¹ to within the 8 to 11 atom range, ie. C₇₋₁₀ alkoxy and alkanoyloxy and C₈₋₁₁ alkyl and alkanoyl. Thus, were R¹ to represent an alkoxy-substituted alkyl group, the alkyl group and alkoxy group are desirably selected to provide a total chain length not in excess of 11, and most preferably in the 7-10 range. In embodiments of the invention, the alkyl, alkoxy, alkanoyl or alkanoyloxy chain of R¹ is substituted by an C₁₋₄alkyl, or hydroxyl group. In specific embodiments of the invention, the R¹ chain is substituted with one or two methyl groups or a hydroxyl group.

The alkyl, alkoxy, alkanoyl or alkanoyloxy chain constituting R¹ can be interrupted by replacement of one or more carbon atoms within the chain with a heteroatom such as nitrogen, oxygen or sulfur. The total chain length of R¹ is maintained in the 8-11 atom range. In embodiments of the invention, the alkyl, alkoxy, alkanoyl or alkanoyloxy chain of R¹ is interrupted by the replacement of a carbon atom with an oxygen atom. In specific embodiments, R¹ represents the group 6-ethyloxy-hexyloxy or 4-butyloxybutyloxy.

R² and R³ are selected independently from H, and C₁₋₃alkyl such as methyl and ethyl. In embodiments of the invention, at least one of R² and R³ is H. In preferred embodiments of the invention, both R² and R³ are H.

R⁴ is selected independently from H, C₁₋₄alkyl, aryl such as phenyl and arylC₁₋₄alkyl such as benzyl and phenethyl. In specific embodiments of the invention, R⁴ is H.

Particular compounds of formula (I) include:
3-(2-aminoethyl)-5-heptyloxyindole
3-(2-aminoethyl)-5-octyloxyindole
3-(2-aminoethyl)-5-nonyloxyindole
3-(2-aminoethyl)-5-decyloxyindole
3-(2-aminoethyl)-5-(7,7-dimethyloctyloxy)indole
3-(2-aminoethyl)-5-(4-butyloxybutyloxy)indole
3-(2-aminoethyl)-5-(6-ethyloxyhexyloxy)indole
3-(2-aminoethyl)-5-(8-hydroxyoctyloxy)indole
and (N-methyl-2-aminoethyl)- and (N,N-dimethyl-2-aminoethyl)-analogs thereof;

3-(2-aminoethyl)-5-octanoylindole
3-(2-aminoethyl)-5-nonanoylindole
3-(2-aminoethyl)-5-decanoylindole
3-(2-aminoethyl)-5-undecanoylindole
and (N-methyl-2-aminoethyl)- and (N,N-dimethyl-2-aminoethyl)-analogs thereof; and

3-(2-aminoethyl)-5-heptanoyloxyindole
3-(2-aminoethyl)-5-octanoyloxyindole
3-(2-aminoethyl)-5-nonanoyloxyindole
3-(2-aminoethyl)-5-decanoyloxyindole
and (N-methyl-2-aminoethyl)- and (N,N-dimethyl-2-aminoethyl)-analogs thereof.

Acid addition salts of the compounds of formula (I) include for example those formed with inorganic acids e.g.hydrochloric, sulphuric or phosphoric acids and organic acids e.g. succinic, maleic, acetic or fumaric acid. Other non-pharmaceutically acceptable salts e.g. oxalates, may be used for example in the isolation of compounds of formula (I) for reagent use, or for subsequent conversion to a pharmaceutically acceptable acid addition salt. Also included within the scope of the invention are solvates and hydrates of the invention.

The conversion of a given compound salt to a desired compound salt is achieved by applying standard techniques, in which an aqueous solution of the given salt is treated with a solution of base e.g. sodium carbonate or potassium hydroxide, to liberate the free base which is then extracted into an appropriate solvent, such as ether. The free base is then separated from the aqueous portion, dried, and treated with the requisite acid to give the desired salt.

It will be appreciated that certain compounds of formula (I) for example where R¹ is substituted C₈₋₁₁alkyl, C₇₋₁₀alkoxy, C₈₋₁₁alkanoyl or C₇₋₁₀alkanoyloxy, may contain an asymmetric centre. Such compounds will exist as two (or more) optical isomers (enantiomers). Both the pure enantiomers and the racemic mixtures (50% of each enantiomer), as well as unequal mixtures of the two, are included within the scope of the present invention. Further, all diastereomeric forms possible (pure enantiomers and mixtures thereof) are within the scope of the invention.

The compounds of the present invention can be prepared by processes analogous to those known in the art. The present invention therefore provides, in a further aspect, a process for the preparation of a compound of formula (I) or a salt, solvate or hydrate thereof, which comprises:
(a) in the case where R¹ is C₇₋₁₀alkoxy and R² and R³ are H,
   deprotecting by treatment with a suitable cleaving agent an intermediate of the structure (II): 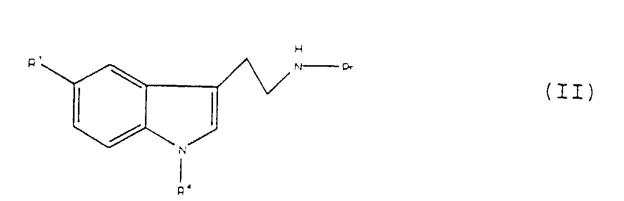 wherein R⁴ are as defined above and Pr represents a protecting group. In a preferred embodiment, Pr represents acetyl or trifluoroacetyl, and the cleaving agent is an inorganic acid such as HCl or a base such as KOH.

The protected structure (II) itself can be prepared by the steps of:
(i) obtaining an intermediate of structure (III): 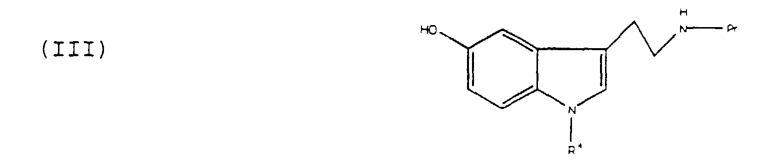 wherein R⁴ and Pr are as just defined; and then,
(ii) reacting the intermediate of structure (III) with the corresponding alkylhalide.

Structure (III) can be obtained by debenzylating the corresponding 5-benzyloxy structure, itself obtained by amidating the 5-benzyloxy structure to incorporate the protecting group, Pr.

To generate products in which both R² and R³ are alkyl groups, synthesis can proceed by (i) obtaining an intermediate of structure (IV) 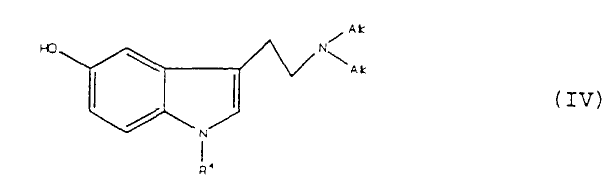 and then (ii) reacting the intermediate of structure (IV) with the appropriate alkylhalide in the case where R¹ is an alkoxy group or with the appropriate alkanoylhalide in the case where R¹ is an alkanoyloxy group.

As an alternative to the synthetic routes just described, and particularly for the production of compounds in which one or both of R² and R³ are C₁₋₄alkyl, compounds of Formula I can be prepared by the steps of obtaining an intermediate of structure (V) 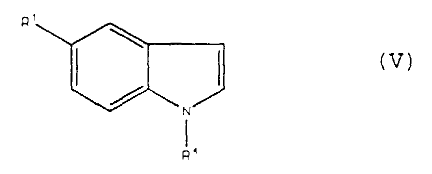 wherein R¹ is an alkyl or alkoxy and R⁴ is defined above, and then elaborating to the corresponding aminoethyl or substituted amino ethyl. This elaboration can be achieved using standard procedures common to the art, for instance, by reaction of intermediate (V) with oxalyl chloride and subsequent amidation by reaction with NHR²R³. Reduction of the resulting glyoxylic amide with a suitable reducing agent, such as lithium aluminum hydride, will provide the desired compound of Formula (I).

To generate compounds wherein one or both of R² and R³ are C₁₋₄ alkyl, and R¹ is an alkanoyl or alkanoyloxy, the intermediate (VI) is prepared from 5-hydroxy indole which is O-protected and reacted with oxalyl chloride and subsequently amidated by reacting with NHR²R³. Reduction of the resulting glyoxylic amide with a suitable reducing agent, such as lithium aluminum hydride, provides the intermediate compound (VI) 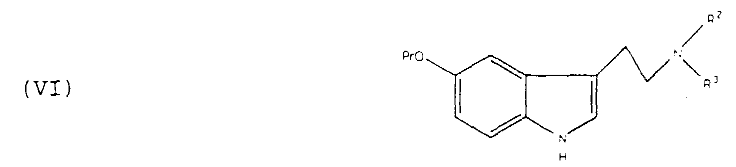 Intermediate (VI) is used to generate N-substituted 3-amino-ethyl compounds wherein R¹ is an alkanoyloxy by deprotecting the 5-hydroxy and subsequently reacting with an acylchloride. Alternatively, (VI) is used to generate the corresponding alkanoyl by reacting with trifluoroacetic anhydride followed by a catalyst, Pd(OAc)₂, carbon monoxide and methanol to yield a 5-methoxycarbonyl intermediate which is converted to the corresponding 5-carboxy compound upon hydrolysis with LiOH in water and methanol. The carboxy intermediate is converted to a Weinreb amide by reacting with N-methylmethoxyamine and a coupling agent EDCI and a catalyst DMAP (dimethylaminopyridine). The Weinreb amide is then reacted with a Grignard reagent to yield the desired N-substituted 3-aminoethyl-5-alkanoyl compound of the invention. The following scheme represents the synthesis of 5-alkanoyl compounds having one or both of R² and R³ substituted with C₁₋₄alkyl.

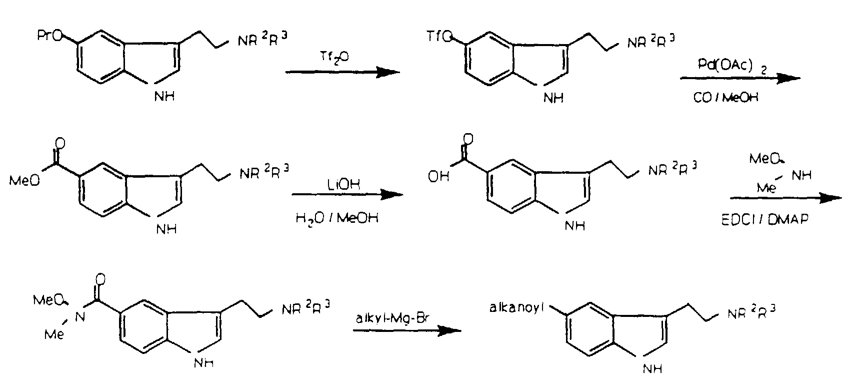

To generate products in which R⁴ is other than H, synthesis can proceed by (i) obtaining a compound of structure (VII) 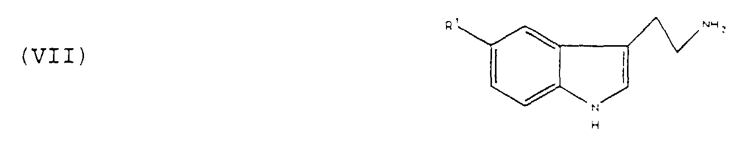 by methods herein described where R¹ is alkoxy, alkyl, alkanoyl or alkanoyloxy; ii) protecting the aminoethyl nitrogen with a suitable protecting group, for example phthalimide to give a compound of structure (VIII) 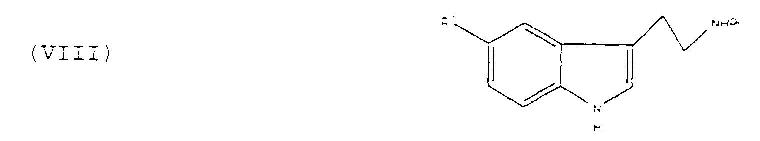 iii) reacting compound (VIII) with the desired alkyl, aryl or arylalkyl halide and then iv) deprotecting the aminoethyl group with an appropriate deprotecting agent, eg. with hydrazine when the protecting group is a phthalimide.

In the case where R¹ is C₈₋₁₁alkyl and R², R³ and R⁴ are hydrogen the following general procedure may be used. The synthetic scheme originates from the position 5 substituent and proceeds to the formation of the indole ring by cyclization followed by activation at the 3 position and subsequent addition of the aminoethyl group. Synthesis begins by obtaining, from commercial sources or by synthesis, aniline substituted at the para position with the desired C₈₋₁₁alkyl group. It will be appreciated that various substitutions may be introduced on the alkyl group by incorporation of the substituent at this stet of the synthesis provided the substituent is stable under subsequent reaction conditions or is protected with an appropriate protecting group. Suitable substituents include hydroxyl, and linear or branched alkyl or alkoxy chains of 4 or fewer carbon atoms such that the total chain length is 8-11 atoms. Further, the C₈₋₁₁alkyl chain may have one or more carbons replaced with a heteroatom such as nitrogen, oxygen or sulfur provided that the heteroatom is stable under the reaction conditions or is appropriately protected.

5-alkyl-substituted 2-alkoxycarbonylindole is obtained by reacting the para-substituted aniline with first sodium nitrite and HCl and then 2-methylacetoacetate and KOH. The 2-alkoxy carbonyl group is removed with a suitable reagent such as KOH and the 3-position is activated with POCl₃ and dimethylformamide (DMF) to give a 5-substituted 3-formyl indole compound. This is converted to a 5-substituted 3-(2-nitro)- ethenyl indole compound by reacting with nitromethane and ammonium acetate. Finally, reaction with lithium aluminum hydride (LiAlH₄) in tetrahydrofuran (THF) yields the desired 3-(2-aminoethyl)-5-alkylindole compound of the invention. The following is a schematic representation of the synthesis of 5-alkyl compounds of the invention:

In the case where R¹ is C₈₋₁₁alkanoyl and R², R³ and R⁴ are hydrogen, the following general procedure described 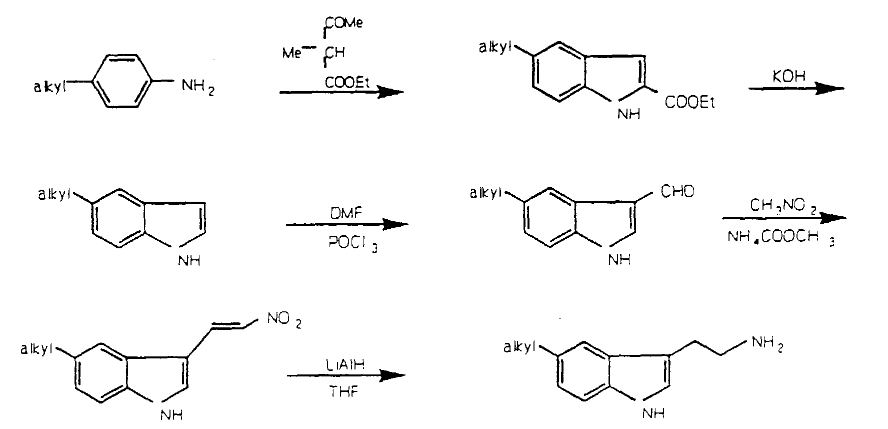 in Strandtmann et al, J. Med. Chem. (1963) 6:719 may be used. Commercially obtained or synthesized C₈₋₁₁alkanoyl- substituted benzenediazonium salt is coupled with 3-carboxy-2-piperidone to give a hydrazone. Cyclization of the hydrazone forms a 6-alkanoyl-1,2,3,4-tetrahydro-1-oxo-β-carboline which is converted to the corresponding 3-(2-aminoethyl)-5-alkanoyl-2-carboxytryptamine by base-catalyzed hydrolysis. Decarboxylation by refluxing with HCl yields the desired 3-(2-aminoethyl)-5-alkanoylindole. It will be appreciated that the alkanoyl- benzenediazonium salt may have substituents on the alkanoyl chain such as C₁₋₄alkyl or C₁₋₄alkoxy. Suitable substituents are those that are stable under coupling, cyclization, hydrolysis and decarboxylation reaction conditions. Further, the alkanoyl chain may have one or more carbons replaced with a heteroatom such as nitrogen oxygen or sulfur provided that the heteroatom is stable under the reaction conditions or is protected by a suitable protecting group common in the art. The following is a schematic representation of the synthesis of 5-alkanoyl compounds of the invention:

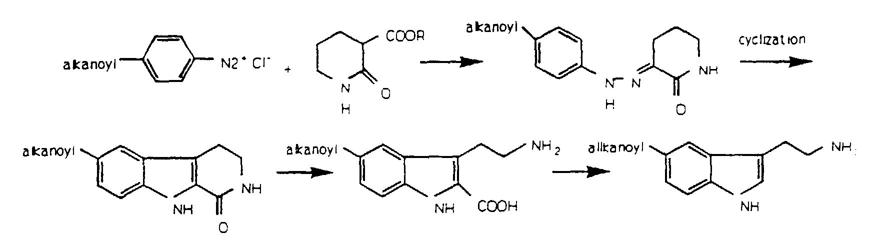

For use as a reagent, the present compounds can be stored in packaged form for reconstitution and use. The compounds, and particularly the preferred nonyloxy compound, can be used to identify 5-HT1D receptors within a population of 5-HT receptors. This can be achieved by incubating the receptors in the presence or absence of the selected compound and then incubating the resulting preparation with a radiolabeled 5-HT ligand, such as ³H-5-HT or ³H-8-OH-DPAT. The 5-HT1D receptors are thus revealed as those receptors that are not labeled when pre-incubated with a selected compound of the present invention. In a preferred embodiment of the invention, this procedure is exploited for the purpose of identifying 5-HT1Dβ receptors and, as ligand, the compound 3-(2-aminoethyl)-5-nonyloxyindole.

In another embodiment of the invention, the compound is provided in labelled form, such as radiolabeled form e.g. labelled by incorporation within its structure of ³H or ¹⁴C or by conjugation to ¹²⁵I. Such radiolabeled forms can be used to directly to distinguish between 5-HT1A and 5-HT1D receptors. Furthermore, radiolabeled forms of the present compounds can be exploited to screen for more potent 5-HT1D ligands, by determining the ability of the test ligand to displace the radiolabeled compound of the present invention.

The sumatriptan-like binding profile of the present compounds indicates their utility as pharmaceuticals that may be useful for the treatment of various conditions in which the use of a 5-HT1Dβ ligand is indicated, such as for the treatment of migraine, cluster headache and portal tension, a condition characterized by increased portal vein blood flow and typically associated with cirrhosis of the liver.

For use in medicine, the compounds of the present invention are usually administered in a standard pharmaceutical composition. The present invention therefore provides in a further aspect pharmaceutical compositions comprising a compound of structure (I) or a pharmaceutically acceptable salt, solvate or hydrate thereof and a pharmaceutically acceptable carrier.

The compounds of the present invention may be administered by an convenient route, for example by oral, parenteral, buccal, sublingual, nasal, rectal or transdermal administration and the pharmaceutical compositions adapted accordingly.

The compounds and their pharmaceutically acceptable salts which are active when given orally can be formulated as liquids, for example syrups, suspensions or emulsions, tablets, capsules and lozenges.

A liquid formulation will generally consist of a suspension or solution of the compound of pharmaceutically acceptable salt in a suitable pharmaceutical liquid carrier for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier, for example aqueous gums, celluloses, silicates or oils and the dispersion or suspension filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilized and then reconstituted with a suitable solvent just prior to administration.

Compositions for nasal administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively, the sealed container may be a unitary dispensing device such as a single dose nasal inhaler or an aerosol dispenser fitted with a metering valve which is intended for disposal after use. Were the dosage form comprises an aerosol dispenser, it will contain a propellant which can be a compressed gas such as compressed air or an organic propellant such as fluorochlorohydrocarbon. The aerosol dosage forms can also take the form of a pump-atomizer.

Compositions suitable for buccal or sublingual administration include tablets, lozenges, and pastilles, wherein the active ingredient is formulated with a carrier such as sugar, acacia, tragacanth, or gelatin and glycerine.

Compositions for rectal administration are conveniently in the form of suppositories containing a conventional suppository base such as cocoa butter.

Preferably, the composition is in unit dose form such as a tablet, capsule or ampoule.

Each dosage unit for oral administration may suitably incorporate from 1 to 250 mg (and for parenteral administration contains preferably from 0.1 to 25 mg) of a compound of the formula (I) or a pharmaceutically acceptable salt thereof calculated as the free base.

The pharmaceutically acceptable compounds of the invention will normally be administered in a daily dosage regimen (for an adult patient) of, for example, an oral dose of from 1 mg to 500 mg, preferably between 10 mg and 400 mg, e.g., between 10 mg and 250 mg, or an intravenous, subcutaneous, or intramuscular dose of between 0.1 mg and 100 mg, preferably between 0.1 mg and 50 mg, e.g., between 1 mg and 25 mg, of the compound of formula (I) or a pharmaceutically acceptable salt, solvate or hydrate thereof calculated as the free base, the compound being administered 1 to 4 times per day. Suitably, the compounds will be administered for a period of continuous therapy, for example for a week or more.

### Example 1 3-(2-Acetamidoethyl)-5-hydroxyindole

The captioned compound served as an intermediate in the preparation of the compounds hereinafter exemplified. To produce this intermediate, a suspension of 5-benzyloxytryptamine (1.9 g, 7.13 mmol) as free base in 10% HCl (30 mL) was treated with NaOAc (20 g) and the solution volume was adjusted to about 80 mL with water. The mixture was allowed to stir at room temperature for 30 min; a few pieces of ice chips were added followed by acetic anhydride (20 mL) and the reaction mixture was allowed to stir for 1 h. The precipitated materials were collected, washed with water (2 x 20 mL), and the solid was recrystallized from CH₂Cl₂/MeOH to give 1.62 g (74%) of the 5-benzyloxy analog of the title compound as a white solid, mp 133-134°C.

A solution of the benzyloxy analog (2,2 g, 7.13 mmol) in absolute EtOH (50 mL) was next treated with Raney Nickel (4.4 g) in a Parr hydrogenation bottle and hydrogenated at 40 p.s.i.[275.79 kPa] overnight. The catalyst was removed by filtration through a Celite pad and the filtrate was concentrated under reduced pressure to give an oil. The oil was purified by column chromatography using a solvent system of CH₂Cl₂/MeOH (90:10) to give 1.48 g (95.1%) of the title intermediate as an oil.

### Example 2 3-(2-aminoethyl)-5-heptyloxyindole hemioxalate

A suspension of 5-benzyloxytryptamine (1.9 g, 7.13 mmol) as free base in 10% HCl (30 mL) was treated with NaOAc (20 g) and the solution volume was adjusted to about 80 mL with water. The mixture was allowed to stir at room temperature for 30 mm; a few pieces of ice chips were added followed by acetic anhydride (20 mL) and the reaction mixture was allowed to stir for 1 h. The precipitated materials were collected, washed with water (2 x 20 mL) and the solid was recrystallized from CH₂Cl₂/hexane to give the acetylated derivative as a white solid.

A solution of this N-acetyl compound (2.2 g, 7.13 mmol) in absolute EtOH (50 mL) was treated with Raney Nickel (4,4 g) in a Parr hydrogenation bottle and hydrogenated at 40 p.s.i. [275.79 kPa] overnight. The catalyst was removed by filtration through a Celite pad and the filtrate was concentrated under reduced pressure to give an oil. The oil was purified by column chromatography using a solvent system of CH₂Cl₂/MeOH (90:10) to give the phenol as an oil.

A stirred mixture o the phenol (0.45 g, 2.07 mmol), 1-bromoheptane (1.75 g, 11.59 mmol), anhydrous K₂CO₃ (0.94 g, 6.83 mmol), and MeOH (7 mL) in 2-butanone (40 mL) was heated at reflux overnight under N₂. After allowing to cool to room temperature, the reaction mixture was filtered and the filtrate was concentrated under reduced pressure to give an oil. A solution of the oil in CH₂Cl₂ (50 mL) was washed successively with 2N NaOH (1 x 30 mL) and water (1 x 30 mL). The organic portion was dried (MgSO₄) and solvent was removed under reduced pressure to give an oil. The oil was purified by column chromatography using a solvent system of CH₂Cl₂/MeOH (90:10) to give the O-alkylated product as an oil. Without further purification, the resulting oil in 2N HCl (10 mL) was heated at reflux for 20 h. After allowing the reaction mixture to cool to room temperature, 2N NaOH (20 mL) was added and the reaction mixture was extracted with CH₂Cl₂ (2 x 30 mL). The combined organic portions were washed with water (1 x 30 mL), dried (MgSO₄), and the solvent was removed under reduced pressure to give an oil. The resulting oil was purified by column chromatography using a solvent system of CH₂Cl₂/MeOH (90:10). The combined fractions from the column were evaporated under reduced pressure to give 3-(2-aminoethyl)-5-heptyloxyindole as an oil. The oil in anhydrous Et₂O (5 mL) was added to a saturated ethereal solution of oxalic acid. The resultant salt was collected by filtration, washed with anhydrous Et₂O (2 x 10 mL), and recrystallized from MeOH/Et₂O to give a white solid, mp 192-194°C. Anal.
(C₃₄H₅₂N₄O₂.C₂H₂O₄.0.25H₂O): C,H,N.

### Example 3 3-(2-aminoethyl)-5-octyloxyindole oxalate

In the manner described in example 2, but using 1-bromooctane in place of 1-bromoheptane, there was produced 3-(2-aminoethyl)-5-octyloxyindole oxalate (white solid, mp 136-139°C. Anal. (C₁₈H₂₈N₂O)). Similarly, 3-(2-aminoethyl)-5-decyloxyindole was produced from 1-bromodecane.

### Example 4 3-(2-aminoethyl)-5-nonyloxyindole oxalate

The intermediate produced as described in Example 1 (0.45 g, 2.07 mmol) was mixed under stirring with 1-bromononane (1.75 g, 11.59 mmol), anhydrous K₂CO₃ (0.94 g, 6.83 mmol), and MeOH (7 mL) in 2-butanone (40 mL) and was heated at reflux overnight under N₂. After allowing to cool to room temperature, the reaction mixture was filtered and the filtrate was concentration under reduced pressure to give an oil. A solution of the oil in CH₂Cl₂ (50 mL) was washed successively with 2N NaOH (1 x 30 mL) and water (1 x 30 mL). The organic portion was dried (MgSO₄) and solvent was removed under reduced pressure to give an oil. The oil was purified by column chromatography using a solvent system of CH₂Cl₂/MeOH (90:10) to give 0.53 g of the O-alkylated product as an oil. Without further purification, the resulting oil in 2N HCl (10 mL) was heated at reflux for 20 h. After allowing the reaction mixture to cool to room temperature, 2N NaOH (20 mL) was added and the reaction mixture was extracted with CH₂Cl₂ (2 x 30 mL). The combined organic portions were washed with water (1 x 30 mL), dried (MgSO₄), and the solvent was removed under reduced pressure to give an oil. The resulting oil was purified by column chromatography using a solvent system of CH₂Cl₂/MeOH (90:10). The combined fractions from the column were evaporated under reduced pressure to give 0.16 g (31%) of the free base of the title compound as an oil. The oil was dissolved in anhydrous Et₂O (5 mL) and added to a saturated ethereal solution of oxalic acid. The resultant oxalate salt was collected by filtration, washed with anhydrous Et₂O (2 x 10 mL), and recrystallized from MeOH/Et₂O to give the title salt compound as a white solid, mp 148-150°C,
Anal. (C₂₁H₃₂N₂O₅):C,H,N.

### Example 5 3-(2-aminoethyl)-5-(8-hydroxyoctyloxy)-indole hydrochloride

To a stirred mixture of serotonin creatinine sulfate monohydrate (1 g, 2.58 mmol), K₂CO₃ (0.712 g, 5.16 mmol) in H₂O (15 mL) was added di-tert-butyl-dicarbonate (0.56 g, 2.58 mmol). The resulting mixture was stirred for 24 h at room temperature. The reaction mixture was extracted into EtOAc (3 x 16 mL) and washed with H₂O (11 mL), 5% HCl (11 mL) and brine solution (20 mL). The organic portion was dried (MgSO₄) and evaporated to dryness to give 0.69 g (96%) of N-t-BOC-serotonin as yellow/brown foam: mp 52-54° C.

A mixture of N-t-BOC-serotonin (0.69 g, 2.50 mmol), 1-bromo-8-tetrahydropyranyloxyoctane (0.73 g, 2.50 mmol) and K₂CO₃ (0.66 g, 4.56 mmol) in acetonitrile (9 mL) was heated to reflux for 24 h under N₂ with stirring. After cooling to room temperature the solid was removed by filtration and solvent evaporated under reduced pressure to give an oil. The oil was purified by flash column chromatography using EtOAc/Hexane (1:3) as the eluent. The product (0.82 g, 67%) was collected as a yellow oil and triturated with hexane to give 3-(N-t-BOC-2-aminoethyl)-5-(8-tetrahydropyranyloxyoctyloxy)indole, yellow solid, mp 56-58° C.

A mixture of 3-(N-t-BHOC-2-aminoethyl)-5-(8-tetrahydropyranyloxyoctyl)indole (100 mg, 0.2 mmol) and pyridinium p-toluenesulfonate (5.1 mg, 0.02 mmol) in 2 mL EtOH was heated at 55° C for 5 h. The solvent was removed under reduced pressure and purified by column chromatography using a solvent system of EtOAc/Hexane (1:3) to afford 70 mg (85%) of 3-(N-t-BOC-2-aminoethyl)-5-(8-hydroxyoctyloxy)indole as an oil.

To a mixture of 3-(N-t-BOC-2-aminoethyl)-5-(8-hydroxyoctyloxy)indole (70 mg, 0.17 mmol) in dry Et₂O (2 mL) was added an ethereal of 3M HCl (10 mL). The mixture was stirred for 5 h. The precipitated product was filtered and washed with Et₂O. The salt was recrystallized from absolute EtOH/Et₂O to afford 30mg (51%) of the title salt, mp 180-182°C. Anal. (C₁₈H₂₈N₂O₂.HCl): C, H, N.

### Example 6 3-(2-aminoethyl)-5-(7-methyloctyloxy)-indole hydrochloride

A mixture of N-t-BOC-serotonin (276 mg, 1 mmol), 1-bromo-7-methyloctane (310 mg, 1.5 mmol) and K₂CO₃ (207 mg, 1.5 mmol) in acetonitrile (20 mL) was refluxed for 24 h under N₂ with stirring. After cooling to room temperature the solid was removed by filtration and solvent evaporated under reduced pressure to give an oil. The oil was purified by column chromatography (25% EtOCAc/hexane) to give 2.93 mg (73%) of 3-(N-t-BOC-2-aminoethyl)-5-(7-methyloctyloxy)indole as an oil.

To a solution of 3-(N-t-BOC-2-aminoethyl)-5-(7-methyloctyloxy) indole (403 mg, 1mnol) in EtOAc (5 mL) was added 3N HCl in EtOAc (15 mL). The mixture was stirred for 2 h. The precipitated product was filtered and washed with EtOAc and Et₂O to give 332 mg (70%) of 3-(2-aminoethyl)-5-(7-methyloctyloxy)indole hydrochloride. Analytical sample was recrystallized from MeOH/Et₂O: mp 193-195°C (dec).
Anal. calcd. for (C₁₉H₃₀N₂O.HCl); C,H,N.

### Example 7 3-(2-aminoethyl)-5-(7,7-dimethyloctyloxy)-indole hydrochloride

A mixture of N-t-BOC-serotonin (276 mg, 1 mmol), 1-bromo-7,7-dimethyloctane (310 mg, 1.5 mmol) and K₂CO₃ (207 mg, 1.5 mmol) in acetonitrile (20 mL) was refluxed for 24 h under N₂ with stirring. After cooling to room temperature the solid was removed by filtration and solvent evaporated under reduced pressure to give an oil. The oil was purified by column chromatography (25% EtOCAc/hexane) to give 3-(N-t-BOC-2-aminoethyl)-5-(7,7-dimethyloctyloxy)indole as an oil.

To a solution of 3-(N-t-BOC-2-aminoethyl)-5-(7,7-dimethyloctyloxy)indole (403 mg, 1mmol) in EtOAc (5 mL) was added 3N HCl in EtOAc (15 mL). The mixture was stirred for 2 h. The precipitated product was filtered and washed with EtOAc and Et₂O to give (62%) 3-(2-aminoethyl)-5-(7,7-dimethyloctyloxy)indole hydrochloride. Analytical sample was recrystallized from MeOH/Et₂O: mp 173-175°C
Anal. calcd. for (C₂₀H₃₃N₂OCl . 0.5H₂O)

### Example 8 3-(2-aminoethyl)-5-(6-ethyloxyhexyloxy)-indole hydrochloride

A mixture of N-t-BOC-serotonin (276 mg, 1 mmol), 1-bromo-6-ethyloxyhexane (310 mg, 1.5 mmol) and K₂CO₃ (207 mg, 1.5 mmol) in acetonitrile (20 mL) was refluxed for 24 h under N₂ with stirring. After cooling to room temperature the solid was removed by filtration and solvent evaporated under reduced pressure to give an oil. The oil was purified by column chromatography (25% EtOCAc/hexane) to give 3-(N-t-BOC-2-aminoethyl)-5-(6-ethyloxyhexyloxy)indole as an oil.

To a solution of 3-(N-t-BOC-2-aminoethyl)-5-(6-ethyloxyhexyloxy)indole (403 mg, 1mmol) in EtOAc (5 mL) was added 3N HCl in EtOAc (15 mL). The mixture was stirred for 2 h. The precipitated product was filtered and washed with EtOAc and Et₂O to give (74%) 3-(2-aminoethyl)-5-(6-ethyloxyhexyloxy)indole hydrochloride.
Analytical sample was recrystallized from MeOH/Et₂O: mp 179-181°C
Anal. calcd. for (C₁₈H₂₉N₂O₂Cl)

### Example 9 3-(2-aminoethyl)-5-(4-butyloxybutyloxy)-indole hydrochoride

A mixture of N-t-BOC-serotonin (276 mg, 1 mmol), 1-bromo-4-butyloxybutane (310 mg, 1.5 mmol) and K₂CO₃ (207 mg, 1.5 mmol) in acetonitrile (20 mL) was refluxed for 24 h under N₂ with stirring. After cooling to room temperature the solid was removed by filtration and solvent evaporated under reduced pressure to give an oil. The oil was purified by column chromatography (25% EtOCAc/hexane) to give 3-(N-t-HOC-2-aminoethyl)-5-(4-butyloxybutyloxy)indole as an oil.

To a solution of 3-(N-t-BOC-2-aminoethyl)-5-(4-butyloxybutyloxy)indole (403 mg, 1mmol) in EtOAc (5 mL) was added 3N HCl in EtOAc (15 mL). The mixture was stirred for 2 h. The precipitated product was filtered and washed with EtOAc and Et₂O to give (80%) 3-(2-aminoethyl)-5-(4-butyloxybutyloxy)indole hydrochloride.
Analytical sample was recrystallized from MeOH/Et₂O: mp 174-176°C
Anal. calcd. for (C₁₈H₃₀N₂O₂Cl . 0.25H₂O)

### Example 10

Also as described in example 2, but with appropriate selection of alkanoylhalide, there are produced the following ester compounds:
(i) 3-(2-aminoethyl)-5-heptanoyloxyindole, from heptanoylchloride;
(ii) 3-(2-aminoethyl)-5-octanoyloxyindole, from octanoylchloride;
(iii) 3-(2-aminoethyl)-5-nonanoyloxyindole, from nonanoylchloride;
(iv) 3-(2-aminoethyl)-5-decanoyloxyindole, from decanoylchloride;

### Example 11 Comparison of Binding Affinities and Selectivities

The compounds of Examples 2-10 were evaluated using cell types receptive specifically to 5-HT1Dβ ligands and to 5-HT1A ligands. The assay protocol generally entailed the incubation of membranes prepared from cells expressing the 5-HT1A receptor and cells expressing the 5-HT1Dβ receptor, with ³H-8-OH-DPAT or ³H-5HT, respectively. Increasing levels of the test compound were incubated with the radioligand and the membrane homogenates prepared from the recombinant cells. After a 15 minute incubation at 37°C, the incubation was terminated by vacuum filtration. The filters were washed with buffer and the filters were counted for radioactivity using liquid scintillation spectrometry. The affinity of the test compound for 5-HT1A or 5HT1Dβ receptor was determined by computer-assisted analysis of the data and determining the amount of the compound necessary to inhibit 50% of the binding of the radioligand to the receptor. Concentrations ranging from 10⁻¹¹M to 10⁻⁵M of the test compound (Example 2) were evaluated. For comparison, sumatriptan, and other compounds structurally related to the test compound were also evaluated. The results are presented in Table 1 below, with reference to the Formula I structure in which R², R³ and R⁴ are each H:

**Table 1**

| R¹ | Compound | 5-HT1Dβ (Ki, nM) | 5-HT1Dβ/5HT1A |
|---|---|---|---|
| H | serotonin | 4.0 | 1 |
| CH₃(CH₂)₆O- | Example 2 | 1.0 | 38 |
| CH₃(CH₂)₇O- | Example 3 | 3.8 | 13 |
| CH₃(CH₂)₈O- | Example 4 | 1.2 | 260 |
| HOCH₂(CH₂)₇O- | Example 5 | 0.3 | 119 |
| (CH₃)₂CH(CH₂)₆O- | Example 6 | 14 | 85 |
| (CH₃)₃C(CH₂)₆O- | Example 7 | 2.3 | 400 |
| CH₃CH₂O(CH₂)₆O- | Example 8 | 0.4 | 180 |
| CH₃(CH₂)₃O(CH₂)₄O- | Example 9 | 2.5 | 30 |
| CH₃(CH₂)₁₀O- | reference | 21 | 42 |
| - | sumatriptan | 5.5 | 60 |

As the tabulated results reveal, either one or both of binding affinity and selectivity for the 5-HT1Dβ receptor are enhanced through extension of the 5-position chain.

To determine the agonist activity of the nonyl compound of Example 4 at the 5-HT1Dβ receptor, the compound was applied in varying concentrations (10⁻¹² to 10⁻⁶M) to 24-well plates containing cultures of 5-HT1Dβ-presenting CHO cells exposed to forskolin, a drug that non-specifically stimulates the enzyme adenylate cyclase. The potency of the compound in inhibiting forskolin-stimulated adenylate cyclase activity was determined by measuring the decrease in cAMP in the wells after 10 minute exposure to various concentrations of the compound. CAMP levels were determined using a radioimmunoassay kit containing antibody specific for cAMP and radioiodinated cAMP. The amount of the compound that inhibited 50% of the forskolin-stimulated adenylate cyclase activity was determined from the inhibition curve data subjected to computer assisted analysis (Figure 1). The effect of 10⁻⁶ 5-HT was assayed in the same experiment for comparison purposes. The results of the assays indicate clearly that the nonyl compound is a potent, full agonist at the 5HT1Dβ receptor.

Using the functional assay just described, the activities of further compounds noted in the following table, were determined in a like manner:

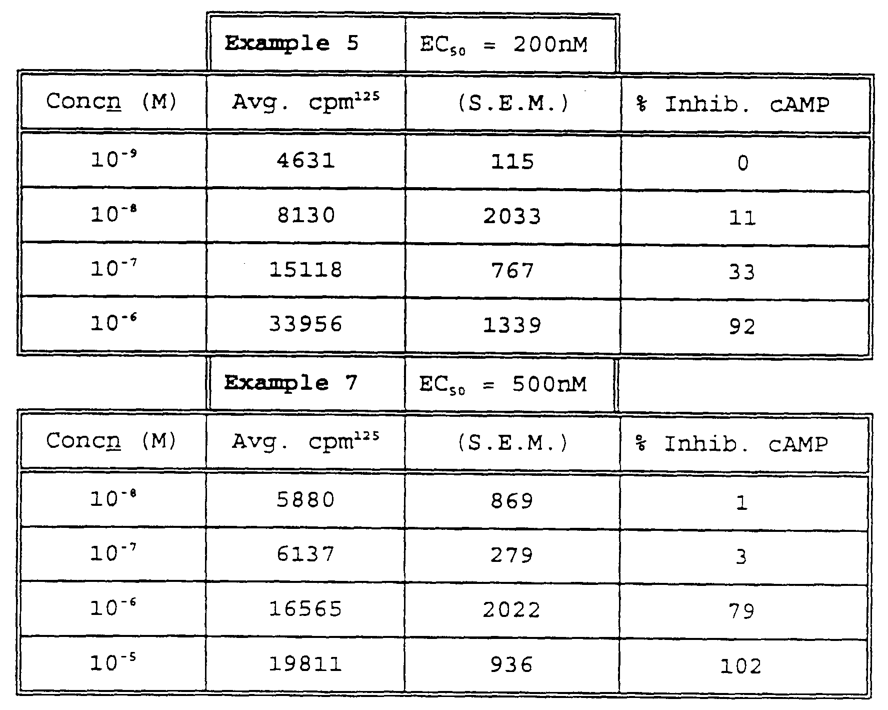

The reference compound identified in Table 1 (R¹ = CH₃(CH₂)₁₀O-) was also evaluated in this functional assay and found to have a much inferior agonist activity (EC₅₀ = 3500nM). Such an activity result is predictable from the binding data presented in Table 1, and further supports the finding that, for optimum bioactivity, the 5'-substituent should have a chain length in the range from 8 to 11 atoms.

## Claims

1. The use for the preparation of a pharmaceutical composition of a compound of the formula: 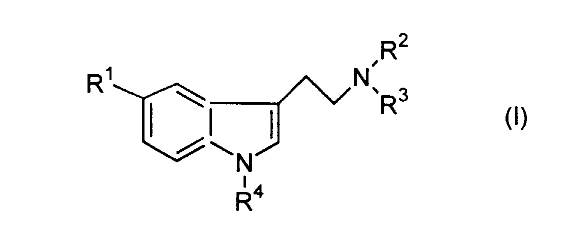 wherein
R¹ represents a chain selected from C₈₋₁₁alkyl, C₇₋₁₀alkoxy, C₈₋₁₁alkanoyl and C₇₋₁₀alkanoyloxy wherein said chain is optionally substituted by hydroxyl, C₁₋₄alkyl or C₁₋₄alkoxy and wherein one of the intervening carbons of said chain is optionally replaced with a heteroatom selected from oxygen, nitrogen and sulfur;
R² and R³ each independently represent H or C₁₋₃alkyl; and
R⁴ represents H, C₁₋₄alkyl, aryl or aryl C₁₋₄alkyl.

2. The use according to claim 1, wherein in the compound of formula (I) R¹ is linear C₇₋₁₀alkoxy optionally substituted by hydroxyl, C₁₋₄alkyl or C₁₋₄alkoxy, wherein one of the intervening carbons of the chain is optionally replaced with an oxygen atom.

3. The use according to claim 1 or claim 2, wherein in the compound of formula (I) R¹ is selected from nonyloxy; heptyloxy; octyloxy; 7,7-dimethyl-octyloxy; 4-butyloxybutyloxy; 6-ethyloxyhexyloxy; and 8-hydroxy-octyloxy.

4. The use according to claim 3, wherein in the compound of formula (I) R¹ is selected from 7,7-dimethyloctyloxy; 6-ethyloxy-hexyloxy; 8-hydroxyoctyloxy; and nonyloxy.

5. The use according to any one of claims 1 to 4, wherein in the compound of formula (I) R⁴ is H.

6. The use according to any one of claims 1 to 5, wherein in the compound of formula (I) R² is H.

7. The use according to any one of claims 1 to 6, wherein in the compound of formula (I) R³ is H.

8. The use according to any one of claims 1 to 7, wherein in the compound of formula (I) R², R³ and R⁴ are H and R¹ is selected from nonyloxy; heptyloxy; octyloxy; 7-methyloctyloxy; 7,7-dimethyl-octyloxy; 4-butyloxy-butyloxy; 6-ethyloxy-hexyloxy; and 8-hydroxy-octyloxy and wherein said compound incorporates a radioactive atom.

9. A pharmaceutical composition, comprising a pharmaceutically acceptable carrier and a compound of formula (I) as defined in any one of claims 1 to 8.

10. A compound of the formula (I) as defined in any one of claims 1 to 8, subject to the proviso that, when R⁴ is hydrogen and one of R² and R³ is hydrogen and the other of R² and R³ is ethyl, then R¹ is not n-octyloxy.

## Patentansprüche

1. Die Verwendung einer Verbindung der Formel 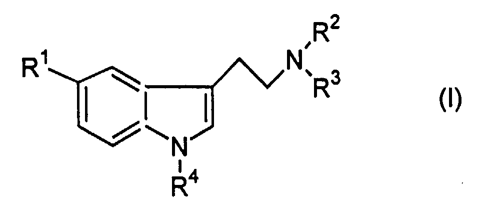 worin
R¹ eine unter C₈₋₁₁-Alkyl, C₇₋₁₀-Alkoxy, C₈₋₁₁-Alkanoyl und C₇₋₁₀-Alkanoyloxy ausgewählte Kette darstellt, die wahlweise durch Hydroxyl, C₁₋₄-Alkyl oder C₁₋₄-Alkoxy substituiert ist und in der einer der mittelständigen Kohlenstoffe wahlweise durch ein unter Sauerstoff, Stickstoff und Schwefel ausgewähltes Heteroatom ersetzt ist;
R² und R³ jeweils unabhängig H oder C₁₋₃-Alkyl darstellen; und
R⁴ H, C₁₋₄-Alkyl, Aryl oder Aryl-C₁₋₄-alkyl darstellt,
für die Herstellung einer pharmazeutischen Zusammensetzung.

2. Die Verwendung nach Anspruch 1, bei der in der Verbindung der Formel (I) R¹ ein wahlweise durch Hydroxyl, C₁₋₄-Alkyl oder C₁₋₄-Alkoxy substituiertes, lineares C₇₋₁₀-Alkoxy ist, bei dem eins der mittelständigen Kohlenstoffe der Kette wahlweise durch ein Sauerstoffatom ersetzt ist.

3. Die Verwendung nach Anspruch 1 oder Anspruch 2, bei der in der Verbindung der Formel (I) R¹ unter Nonyloxy, Heptyloxy, Octyloxy, 7,7-Dimethyl-octyloxy, 4-Butyloxy-butyloxy, 6-Ethyloxyhexyloxy und 8-Hydroxy-octyloxy ausgewählt ist.

4. Die Verwendung nach Anspruch 3, bei der in der Verbindung der Formel (I) R¹ unter 7,7-Dimethyloctyloxy. 6-Ethyloxy-hexyloxy, 8-Hydroxyoctyloxy und Nonyloxy ausgewählt ist.

5. Die Verwendung nach einem der Ansprüche 1 bis 4, bei der in der Verbindung der Formel (I) R⁴ H ist.

6. Die Verwendung nach einem der Ansprüche 1 bis 5, bei der in der Verbindung der Formel (I) R² H ist.

7. Die Verwendung nach einem der Ansprüche 1 bis 6, bei der in der Verbindung der Formel (I) R³ H ist.

8. Die Verwendung nach einem der Ansprüche 1 bis 7, bei der in der Verbindung der Formel (I) R², R³ und R⁴ H sind und R¹ unter Nonyloxy, Heptyloxy, Octyloxy, 7-Methyloctyloxy, 7,7-Dimethyloctyloxy, 4-Butyloxy-butyloxy, 6-Ethyloxy-hexyloxy und 8-Hydroxyoctyloxy ausgewählt ist, und bei der die Verbindung ein radioaktives Atom enthält.

9. Eine pharmazeutische Zusammensetzung mit einem pharmazeutisch zulässigen Träger und einer Verbindung der Formel (I), wie sie in einem der Ansprüche 1 bis 8 definiert ist.

10. Eine Verbindung der Formel (I), wie sie in einem der Ansprüche 1 bis 8 definiert ist, unter der Bedingung, daß R¹ nicht n-Octyloxy ist, wenn R⁴ Wasserstoff ist und einer von R² und R³ Wasserstoff und der andere von R² und R³ Ethyl ist.

## Revendications

1. Utilisation pour la préparation d'une composition pharmaceutique d'un composé de formule: 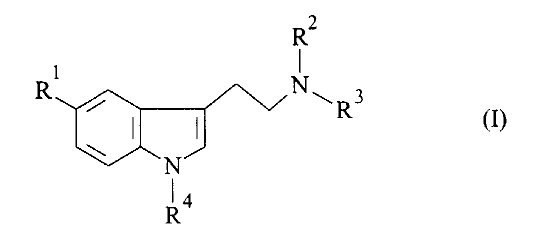 dans laquelle
R¹ représente une chaîne choisie parmi alkyle en C₈₋₁₁, alcoxy en C₇₋₁₀, alcanoyle en C₈₋₁₁, et alcanoyloxy en C₇₋₁₀, dans laquelle ladite chaîne est éventuellement substituée par hydroxy, alkyle en C₁₋₄ ou alcoxy en C₁₋₄ et dans laquelle l'un des atomes de carbone intermédiaires de ladite chaîne est éventuellement remplacé par un hétéroatome choisi parmi l'oxygène, l'azote et le soufre;
R² et R³ représentent chacun indépendamment H ou alkyle en C₁₋₃; et
R⁴ représente H ou alkyle en C₁₋₄, aryle ou aryl(alkyle en C₁₋₄).

2. Utilisation selon la revendication 1, dans laquelle, dans le composé de formule (I), R¹ est alcoxy linéaire en C₇₋₁₀ éventuellement substitué par hydroxy, alkyle en C₁₋₄ ou alcoxy en C₁₋₄, dans laquelle l'un des atomes de carbone intermédiaires de la chaîne est éventuellement remplacé par un atome d'oxygène.

3. Utilisation selon la revendication 1 ou la revendication 2, dans laquelle, dans le composé de formule (I), R¹ est choisi parmi nonyloxy, heptyloxy, octyloxy, 7,7-diméthyloctyloxy, 4-butyloxybutyloxy, 6-éthoxyhexyloxy et 8-hydroxyoctyloxy.

4. Utilisation selon la revendication 3, dans laquelle, dans le composé de formule (I), R¹ est choisi parmi 7,7-diméthyloctyloxy, 6-éthoxyhexyloxy, 8-hydroxyoctyloxy et nonyloxy.

5. Utilisation selon l'une quelconque des revendications 1 à 4, dans laquelle, dans le composé de formule (I), R⁴ est H.

6. Utilisation selon l'une quelconque des revendications 1 à 5, dans laquelle, dans le composé de formule (I), R² est H.

7. Utilisation selon l'une quelconque des revendications 1 à 6, dans laquelle, dans le composé de formule (I), R³ est H.

8. Utilisation selon l'une quelconque des revendications 1 à 7, dans laquelle, dans le composé de formule (I), R², R³ et R⁴ sont H et R¹ est choisi parmi nonyloxy, heptyloxy, octyloxy, 7-méthyloctyloxy, 7,7-diméthyloctyloxy, 4-butyloxybutyloxy, 6-éthoxyhexyloxy et 8-hydroxyoctyloxy et dans laquelle ledit composé comprend un atome radioactif.

9. Composition pharmaceutique comprenant un support pharmaceutiquement acceptable et un composé de formule (I) tel que défini dans l'une quelconque des revendications 1 à 8.

10. Composé de formule (I) tel que défini dans l'une quelconque des revendications 1 à 8, à condition que, lorsque R⁴ est hydrogène et l'un des R² et R³ est hydrogène et l'autre des R² et R³ est éthyle, alors R¹ ne soit pas n-octyloxy.
